# EUROPÄISCHE PATENTSCHRIFT

(11) **EP 1 233 961 B1**
(45) Veröffentlichungstag und Bekanntmachung des Hinweises auf die Patenterteilung: **06.07.2005**
(21) Anmeldenummer: 00993257.5
(22) Anmeldetag: 29.11.2000
(51) Int. Cl.: C07D 405/00

(54) **VERFAHREN ZUR HERSTELLUNG VON 5-(1-PIPERAZINYL)-BENZOFURAN-2-CARBOXAMID DURCH ÜBERGANSMETALL-KATALYSIERTE AMINIERUNG**
METHOD FOR PRODUCING 5-(1-PIPERAZINYL)-BENZOFURAN-2-CARBOXAMIDE BY TRANSITION METAL-CATALYZED AMINATION
PROCEDE DE PREPARATION DE 5-(1-PIPERAZINYL)-BENZOFURAN-2-CARBOXAMIDE PAR AMINATION CATALYSEE PAR METAL DE TRANSITION

(30) Priorität: 04.12.1999 DE 19958496
(43) Veröffentlichungstag der Anmeldung: 28.08.2002
(73) Patentinhaber: MERCK PATENT GmbH, 64293 Darmstadt (DE)
(72) Erfinder: BATHE, Andreas, 64283 Darmstadt (DE); EMMERT, Steffen, 64331 Weiterstadt (DE); HELFERT, Bernd, 64372 Ober-Ramstadt (DE); BOETTCHER, Henning, 64287 Darmstadt (DE)
(86) Internationale Anmeldenummer: PCT/EP2000/011980
(87) Internationale Veröffentlichungsnummer: WO 2001/040219

(56) Entgegenhaltungen:
- EP-A- 0 738 722
- EP-A- 0 802 173
- EP-A- 0 846 676
- US-A- 4 210 646
- US-A- 4 814 262
- NISHIYAMA M ET AL: "Synthesis of N-Arylpiperazines from Aryl Halides and Piperazine under a Palladium Tri-tert-butylphosphine Catalyst" TETRAHEDRON LETTERS,NL,ELSEVIER SCIENCE PUBLISHERS, AMSTERDAM, Bd. 39, Nr. 7, 12. Februar 1998 (1998-02-12), Seiten 617-620, XP004106753 ISSN: 0040-4039 in der Anmeldung erwähnt

## Beschreibung

Die Erfindung betrifft ein Verfahren zur Herstellung von 5-(1-Piperazinyl)-benzofuran-2-carboxamid, dadurch gekennzeichnet, daß man
a) 5-Brom-salicylaldehyd in einer Eintopfreaktion zuerst mit einer Verbindung der Formel I

   L-CH₂-COOR¹ I

   worin
   - L: Cl, Br, I oder eine reaktionsfähig veresterte OH-Gruppe und
   - R¹: Alkyl mit 1-6 C-Atomen oder Benzyl bedeuten,
   und anschließend mit Formamid zu 5-L-benzofuran-2-carboxamid (II),
   worin L Cl, Br, I oder eine reaktionsfähig veresterte OH-Gruppe bedeutet,
   umsetzt,
   darauf (II) in einer Übergangsmetall-katalysierten Aminierung mit R²-Piperazin, worin R² H oder eine Aminoschutzgruppe bedeutet, zu einer Verbindung der Formel III 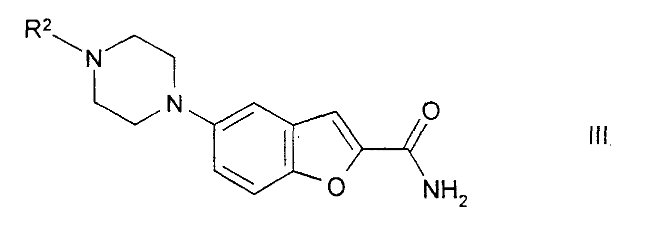 worin R² H oder eine Aminoschutzgruppe bedeutet,
   umsetzt,
   und anschließend, falls R² ≠ H ist, R² abspaltet,
   oder
b) eine Verbindung der Formel IV 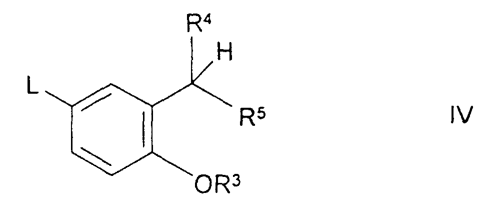 worin
   - L: Cl, Br, I oder eine reaktionsfähig veresterte OH-Gruppe,
   - R³: H oder CH₂R⁶,
   - R⁴ und R⁵: zusammen Carbonyl,
   - R⁶: CN, COOH, COOR⁷ oder CONH₂,
   - R⁷: Alkyl mit 1-6 C-Atomen,
   - n: 2 oder 3
   bedeuten,
   in einer Übergangsmetall-katalysierten Aminierung mit R²-Piperazin, worin R² H oder eine Aminoschutzgruppe bedeutet,
   zu einer Verbindung der Formel V 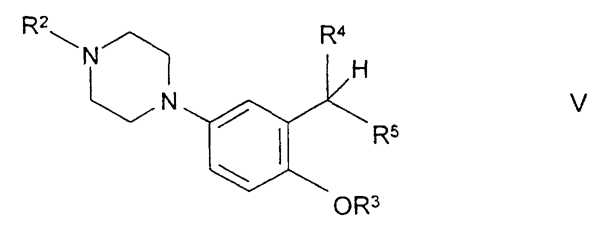 worin
   - R²: H oder eine Aminoschutzgruppe,
   - R³: H oder CH₂R⁶,
   - R⁴ und R⁵: zusammen Carbonyl,
   - R⁶: CN, COOH, COOR⁷ oder CONH₂,
   - R⁷: Alkyl mit 1-6 C-Atomen,
   - n: 2 oder 3
   bedeuten,
   umsetzt,
   anschließend in einer Eintopfreaktion zuerst mit einer Verbindung der Formel I

   L-CH₂-COOR¹ I

   worin
   - L: Cl, Br, I oder eine reaktionsfähig veresterte OH-Gruppe und
   - R¹: Alkyl mit 1-6 C-Atomen oder Benzyl bedeuten,
   und anschließend mit Formamid zu einer Verbindung der Formel III 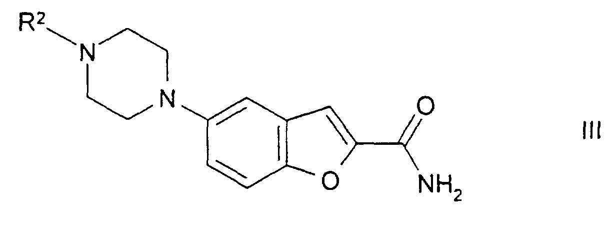 worin R² H oder eine Aminoschutzgruppe bedeutet,
   umsetzt,
   und anschließend, falls R² ≠ H ist, R² abspaltet,
   oder
c) eine Verbindung der Formel V
   worin
   - R²: eine Aminoschutzgruppe,
   - R³: H, CH₂R⁶,
   - R⁴ und R⁵: zusammen Carbonyl,
   - R⁶: CN, COOH, COOR⁷ oder CONH₂,
   - R⁷: Alkyl mit 1-6 C-Atomen,
   - n: 2 oder 3
   bedeuten,
   mit Chloracetamid zu einer Verbindung der Formel III
   worin R² eine Aminoschutzgruppe bedeutet,
   umsetzt,
   und anschließend R² abspaltet,
   und/oder daß man 5-(1-Piperazinyl)-benzofuran-2-carboxamid durch Behandeln mit einer Säure in eines seiner Säureadditionssalze umwandelt.

Gegenstand der Erfindung sind auch die Verbindungen der Formel V 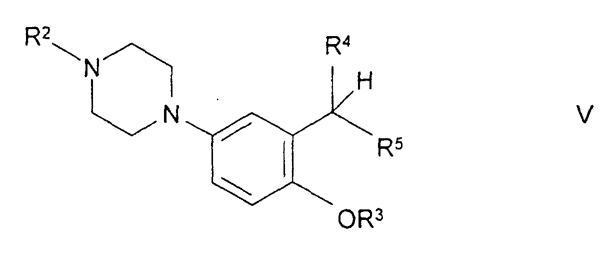 worin
- R²: H oder eine Aminoschutzgruppe,
- R³: H, CH₂R⁶,
- R⁴ und R⁵: zusammen Carbonyl,
- R⁶: CN, COOH, COOR⁷ oder CONH₂,
- R⁷: Alkyl mit 1-6 C-Atomen,
- n: 2 oder 3
bedeuten,
sowie deren Salze und Solvate.

5-(1-Piperazinyl)-benzofuran-2-carboxamid ist ein wichtiges Zwischenprodukt für pharmazeutische Wirkstoffe. Dies ist z.B. beschrieben in DE 19730989, WO 9857953, EP 738722, EP 736525, DE 4414113, DE 4333254 oder DE 4101686.
Benzofuranderivate als Vorstufen sind z.B. auch in DE 19514567 beschrieben.

Bekannt sind Verfahren zur Herstellung von heterocyclischen aromatischen Aminen oder Arylaminen z.B. aus EP 0 802 173, wobei ein Übergangsmetallkatalysator verwendet wird.
Allgemeine Aminierungsreaktionen werden in einem review-Artikel von J.F.Martinez in Angew. Ch. Int. 37, 1998, 2046-2062 beschrieben. Andere Verfahren zur Herstellung von tertiären Arylaminen unter Verwendung eines Katalysators, zusammengesetzt aus einem Trialkylphosphin und Palladium, kennt man aus JP 10-310561 (Application-Kokai), Appl. No. 9-119477 oder JP 11-80346 (Application-Kokai), Appl. No. 9-245218.

Ein Verfahren zur Herstellung von Arylaminen unter Übergangsmetall-Katalyse ist von S.L.Buchwald et al. in US 5,576,460 beschrieben. Ein anderes Verfahren zur Herstellung von aromatischen Aminen aus Chloraromaten in Gegenwart eines Palladiumkatalysators kennt man aus EP 0 846 676, von J.F.Hartwig et al. aus J. Org. Chem. 1999, S. 5575-5580 oder von S.L. Buchwald et al. aus J.A.C.S. 1999, 121, 9550-9561.

In Tetrahedron Letters **39** (1998) 617-620 ist die Synthese von N-Arylpiperazinen aus Arylhaliden und Piperazin unter Übergangsmetall-Katalyse von M. Nishiyama beschrieben.

Überraschenderweise ergaben Untersuchungen im Rahmen der Synthese von Arzneimitteln, welche z.B. in der DE 43 33 254 (EP 0 648 767) beschrieben sind, daß 5-(1-Piperazinyl)-benzofuran-2-carboxamid im Vergleich zum Stand der Technik in wenigstens vergleichbarer oder höherer Gesamtausbeute erhalten werden kann, wobei als entscheidende Vorteile dabei die einfache durchzuführende Reaktion sowie eine dadurch bedingt einfache Produktisolierung zu nennen sind.
Das bedeutet in der Konsequenz auch einen geringeren Lösungsmittel- und Energieverbrauch.

Falls in den Verbindungen der Formeln I, II bzw. IV L eine reaktionsfähige veresterte OH-Gruppe bedeutet, so ist diese vorzugsweise Alkylsulfonyloxy mit 1-6 C-Atomen (bevorzugt Methylsulfonyloxy oder Trifluormethylsulfonyloxy), Arylsulfonyloxy mit 6-10 C-Atomen (bevorzugt Phenyl- oder p-Tolylsulfonyloxy, ferner auch 2-Naphthalinsulfonyloxy) oder auch Fluorsulfonyloxy.

R¹ bedeutet Alkyl oder Benzyl. Dabei hat Alkyl 1, 2, 3, 4, 5 oder 6 C-Atome, vorzugsweise 1, 2, 3 oder 4 C-Atome, insbesonders sind bevorzugt z.B. Methyl oder Ethyl, weiterhin Propyl, Isopropyl, ferner auch Butyl, Isobutyl, sek.-Butyl oder tert.-Butyl.

In den Verbindungen der Formel I bedeutet L vorzugsweise Cl, ferner auch Br.

R² bedeutet H oder eine Aminoschutzgruppe. Besonders bevorzugt bedeutet R² eine Aminoschutzgruppe.
Der Ausdruck "Aminoschutzgruppe" ist allgemein bekannt und bezieht sich auf Gruppen, die geeignet sind, eine Aminogruppe vor chemischen Umsetzungen zu schützen (zu blockieren), die aber leicht entfernbar sind, nachdem die gewünschte chemische Reaktion an anderen Stellen des Moleküls durchgeführt worden ist. Typisch für solche Gruppen sind insbesondere unsubstituierte Acyl-, Aryl-, Aralkoxymethyl- oder Aralkylgruppen. Da die Aminoschutzgruppen nach der gewünschten Reaktion (oder Reaktionsfolge) entfernt werden, ist ihre Art und Größe im übrigen nicht kritisch; bevorzugt werden jedoch solche mit 1-20, insbesondere 1-8 C-Atomen. Der Ausdruck "Acylgruppe" ist im Zusammenhang mit dem vorliegenden Verfahren und den vorliegenden Verbindungen in weitestem Sinne aufzufassen. Er umschließt von aliphatischen, araliphatischen, aromatischen oder heterocyclischen Carbonsäuren oder Sulfonsäuren abgeleitete Acylgruppen sowie insbesondere Alkoxycarbonyl-, Aryloxycarbonyl- und vor allem Aralkoxycarbonylgruppen. Beispiele für derartige Acylgruppen sind Alkanoyl wie Acetyl, Propionyl, Butyryl; Aralkanoyl wie Phenylacetyl; Aroyl wie Benzoyl oder Toluyl; Aryloxyalkanoyl wie Phenoxyacetyl; Alkoxycarbonyl wie Methoxycarbonyl, Ethoxycarbonyl, 2.2,2-Trichlorethoxycarbonyl, BOC (tert.-Butoxycarbonyl), 2-Iodethoxycarbonyl; Aralkyloxycarbonyl wie CBZ (Carbobenzoxycarbonyl, auch "Z" genannt), 4-Methoxybenzyloxycarbonyl, FMOC (9-Fluorenylmethoxycarbonyl); Arylsulfonyl wie Mtr (4-Methoxy-2,3,6-trimethylphenyl-sulfonyl).
R² bedeutet ganz besonders bevorzugt Benzyl oder BOC.

Die Abspaltung einer Aminoschutzgruppe aus einer Verbindung der Formel III erfolgt - je nach der benutzten Schutzgruppe - z.B. mit starken Säuren. zweckmäßig mit TFA (Trifluoressigsäure) oder Perchlorsäure, aber auch mit anderen starken anorganischen Säuren wie Salzsäure oder Schwefelsäure, starken organischen Carbonsäuren wie Trichloressigsäure oder Sulfonsäuren wie Benzol- oder p-Toluolsulfonsäure. Die Anwesenheit eines zusätzlichen inerten Lösungsmittels ist möglich, aber nicht immer erforderlich. Als inerte Lösungsmittel eignen sich vorzugsweise organische, beispielsweise Carbonsäuren wie Essigsäure, Ether wie Tetrahydrofuran oder Dioxan, Amide wie Dimethylformamid, halogenierte Kohlenwasserstoffe wie Dichlormethan, ferner auch Alkohole wie Methanol, Ethanol oder Isopropanol sowie Wasser. Ferner kommen Gemische der vorgenannten Lösungsmittel in Frage. TFA wird vorzugsweise im Überschuß ohne Zusatz eines weiteren Lösungsmittels verwendet. Perchlorsäure in Form eines Gemisches aus Essigsäure und 70 %iger Perchlorsäure im Verhältnis 9 : 1. Die Reaktionstemperaturen liegen zweckmäßig zwischen etwa 0 und etwa 50°. vorzugsweise arbeitet man zwischen 15 und 30°.
Die Gruppe BOC wird bevorzugt mit TFA in Dichlormethan oder mit etwa 3 bis 5 n Salzsäure in Dioxan bei 15-30° abgespalten.
Hydrogenolytisch entfernbare Schutzgruppen (z.B. CBZ oder Benzyl) können z.B. durch Behandeln mit Wasserstoff in Gegenwart eines Katalysators (z.B. eines Edelmetallkatalysators wie Palladium, zweckmäßig auf einem Träger wie Kohle) abgespalten werden. Als Lösungsmittel eignen sich dabei die oben angegebenen, insbesondere z.B. Alkohole wie Methanol oder Ethanol oder Amide wie DMF. Die Hydrogenolyse wird in der Regel bei Temperaturen zwischen etwa 0 und 100° und Drucken zwischen etwa 1 und 200 bar, bevorzugt bei 20-30° und 1-10 bar durchgeführt.
R³ bedeutet vorzugsweise H.
R⁴ und R⁵ bedeuten zusammen Carbonyl.
In den Verbindungen der Formel IV bedeutet Hal vorzugsweise Br.
Die Verbindungen der Formel IV bzw. V können auch in einer dimeren, in entsprechende Salicylaldehyde rückspaltbaren Form vorliegen, wobei L und R² die angegebenen Bedeutungen haben:
R⁷ bedeutet Alkyl. Dabei hat Alkyl 1, 2, 3, 4, 5 oder 6 C-Atome, vorzugsweise 1, 2, 3 oder 4 C-Atome, insbesonders sind bevorzugt z.B. Methyl oder Ethyl, weiterhin Propyl, Isopropyl, ferner auch Butyl, Isobutyl, sek.-Butyl oder tert.-Butyl.

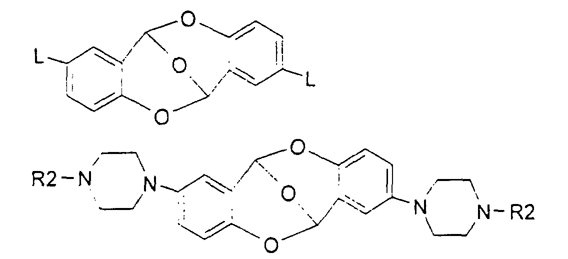

Die Verbindungen der Formel I und IV sind entweder bekannt oder werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

### Verfahrensvariante a)

Die Umsetzung von 5-Brom-salicylaldehyd mit einer Verbindung der Formel I und anschließend mit Formamid verläuft in einem geeigneten inerten Lösungsmittel unter Zusatz einer Base als Eintopfreaktion. Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan, Ether wie Diethylether, Diisopropylether, Tetrahydrofuran (THF) oder Dioxan; Glykolether wie Ethylenglykolmonomethyl- oder - monoethylether (Methylglykol oder Ethylglykol), Ethylenglykoldimethylether (Diglyme); Ketone wie Aceton oder Butanon; Nitrile wie Acetonitril; Sulfoxide wie Dimethylsulfoxid (DMSO); Schwefelkohlenstoff; Nitroverbindungen wie Nitromethan oder Nitrobenzol; gegebenenfalls auch Gemische der genannten Lösungsmittel untereinander.
Die Reaktionszeit liegt je nach den angewendeten Bedingungen zwischen einigen Minuten und 14 Tagen, die Reaktionstemperatur zwischen etwa 0° und 150°, vorzugsweise zwischen 60° und 120°.
Ganz besonders bevorzugt liegt die Reaktionszeit zwischen 4 und 20 Stunden und die Temperatur zwischen 90 und 115°.
Als Basen eignen sich Verbindungen wie z.B. Na-, K-, oder Cs-carbonat.

In einer Eintopfreaktion wird anschließend mit Formamid vorzugsweise in Gegenwart einer organischen Base, vorzugsweise eines Alkalialkoholats, wie z.B. Na-tert.-Butylat, und seines entsprechenden Alkohols, zu 5-Halbenzofuran-2-carboxamid (II) umgesetzt. In (II) bedeutet Hal vorzugsweise Br.
Die Umsetzung erfolgt vorzugsweise bei 0 bis 60°.
Andere Verfahren zu (II) sind z.B. in Bull. Soc. Chim. Fr., 1971; 4329 und von O. Dann et al. in Justus Liebigs Ann. Chem. 1975; 160-194 beschrieben.
Die oben beschriebene Eintopfreaktion verläuft in besserer Ausbeute als die bekannten Umsetzungen.

Die Umsetzung von (II) mit R²-Piperazin zur Verbindung der Formel III verläuft in einem geeigneten inerten Lösungsmittel, einer Base und in Gegenwart eines Übergangsmetallkatalysators.

Als Übergangsmetalle können u.a. PdCl₂ oder Pd (OAc)₂ oder andere Pd²⁺-Derivate eingesetzt werden, welche z.B. mit NaBH₄ oder Phosphinen vorreduziert werden (der Schritt kann bei einem Überschuß an Ligand R₃P entfallen) oder Pd-(O)-Spezies wie z.B. Pd(DBA)₂ oder Pd₂(DBA)₃ (DBA = Dibenzylidenaceton).
Zu diesem Spektrum an Pd-Komplexen kommen entsprechende Ligandenkomplexe des Nickels oder Kupfers.
Ferner sind als Liganden N,N-Diaryl-imidazoliumsalze analog J.Huang et al., Org. Lett. 1, 1999, 1307-1309. einsetzbar.

Die eingesetzten Phosphan- bzw. Aza-/Phosphan-Liganden sind u.a.
Tris-ortho-tolylphosphin
Tricyclohexylphosphin
1-(2-Diphenylphosphino-1-naphthyl)-isochinolin (QUINAP)
1,8-Bis(Dimethylamino-)naphthalin
Phe₂P-CH₂-PPhe₂
insbesondere auch P(tert.-Butyl)₃ = P(t-Bu)₃
1,1'-Bis(diphenylphosphano)ferrocen (DPPF als Komplex DPPFxPdCl₂)
2,2'-Bis(diphenylphosphino)-1,1'-binaphthyl (= BINAP)
(S)-dibutphos = 1-(2-di-tert-butylphasphanyl-phenyl)-ethyl-dimethyl-amine
1-(N,N-Dimethylamino)-1'-(dicyclohexylphosphino)biphenyl
1-(di-t-Butylphosphino)biphenyl
1,1'-Bis(di-t-Butylphosphino)biphenyl
(t-Bu)₂P-(CH₂)ₙ-P(t-Bu)₂ n=1,2,3
(t-Bu)₂P-(CH₂)ₘ-X-(CH₂)ₙ-P(t-Bu)₂ m,n=1,2,3; X = O,.....
oder auch
DB ^{t} PF = 1,1'-bis(di-*tert*-butylphosphino)ferrocene.

Als Lösungsmittel eignen sich z.B. Kohlenwasserstoffe, wie Benzol, Toluol, Xylol; chlorierte Kohlenwasserstoffe wie z.B. Dichlormethan; Ketone wie Aceton, Butanon; Ether wie Tetrahydrofuran (THF) oder Dioxan; Nitrile wie Acetonitril, gegebenenfalls auch Gemische dieser Lösungsmittel untereinander.

Die Reaktionszeit liegt je nach den angewendeten Bedingungen zwischen einigen Minuten und 14 Tagen, die Reaktionstemperatur zwischen etwa 0° und 180°, normalerweise zwischen 30° und 130°.
Als Basen eignen sich z.B. Alkalialkoholate, wie z.B. Na-tert.Butylat.

### Verfahrensvariante b)

Die Umsetzung der Verbindungen der Formel IV mit R²-Piperazin verläuft unter Bedingungen wie unter Variante a) beschrieben.
Gegebenenfalls werden R⁴ und R⁵ in eine Carbonylgruppe überführt.
Die sich anschließende Eintopfreaktion der Verbindung der Formel V mit einer Verbindung der Formel I und anschließend mit Formamid verläuft ebenfalls unter Bedingungen wie oben beschrieben. Auch die Abspaltung von R², falls R² ≠ H, erfolgt unter den beschriebenen Bedingungen.

Eine Base der Formel I bzw. der Formel V kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuren, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und Disulfonsäuren, Laurylschwefelsäure. Salze mit physiologisch nicht unbedenklichen Säuren, z.B. Pikrate, können zur Isolierung und /oder Aufreinigung der Verbindungen der Formel I verwendet werden.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab. trocknet die organische Phase über Natriumsulfat, dampft ein und reinigt durch Chromatographie an Kieselgel und /oder durch Kristallisation.

### Beispiel 1

### 1) Synthese von 5-Brom-benzofuran-2-carboxamid

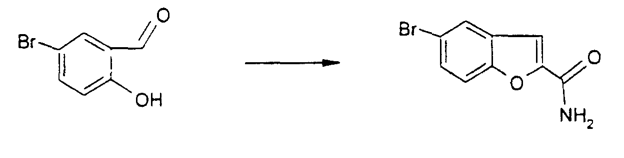

### Durchführung der Reaktion mit Ethylbromacetat:

200 g 5-Brom-2-Hydroxybenzaldehyd werden in 2000 mL NMP unter Rühren gelöst und 144 g Kaliumcarbonat und 175 g Ethylbromacetat zugegeben. Es wird unter Stickstoff für 15 Stunden bei 105 ° gerührt. Die entstehende, orange gefärbte und mit Kristallen durchsetzte Lösung wird auf 25 ° abgekühlt und mit 135 g Formamid versetzt und 30 Minuten nachgerührt. Dann läßt man in 15 Minuten ohne Kühlung 557 mL Natriummethylat (30% in MeOH) zulaufen. Nach 3 Stunden liegt eine bräunliche, mit Kristallen durchsetzte Lösung vor. Sie wird auf 6 Liter VE-Wasser (10 °) gegossen und 30 Miuten nachgerührt. Die Kristalle werden abgesaugt, mit 1 Liter VE-Wasser gewaschen, nochmals mit 4 Liter VE-Wasser angeschlämmt und abgesaugt. Es wird erneut mit 1 Liter VE-Wasser gewaschen. Über Nacht werden die Kristalle bis zur Gewichtskonstanz im Vakuum bei 60 °C getrocknet (Auswaage: 113 g hellbeige Kristalle; Fp. 210 - 213°; CAS 35351-21-4).
Die physik. und spektr. Daten entsprechen den veröffentlichten Angaben in: Rene; Royer; BSCFAS; Bull.Soc.Chim.Fr.; 1971; 4329 und Dann.O. et al.; JLACBF; Justus Liebigs Ann. Chem.; GE; 1975; 160-194.

Mit gleicher Methodik läßt sich bei vergleichbaren Ausbeuten 5-Chlorbenzofuran-2-carboxamid (Fp. 200-202°), 5-Fluor-benzofuran-2-carboxamid und 5-lod-benzofuran-2-carboxamid gewinnen.

### 2) Synthese von 5-(4-Benzyl-1-piperazinyl)-benzofuran-2-carboxamid durch übergangsmetall-kat. Aminierung von 5-Brom-benzofuran-2-carboxamid mit Benzylpiperazin

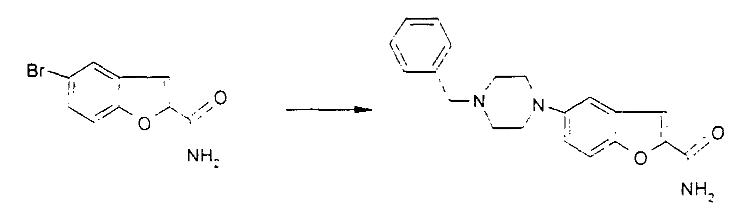

### Beispielhafte Durchführung mit dem Katalysatorsystem Pd(OAc)₂ / P(t-Bu)₃:

Zu einer Suspension von 0,085 g Pd(II)acetat in 150 mL Xylol werden nach 15 min Rühren 0,30 g P(t-Bu)₃, 4,5 g 5-Brom-benzofuran-2-carboxamid, 4,9 g Benzylpiperazin und 5,0 g Na-t-OBu gegeben und unter Schutzgas Stickstoff für 12-18 h auf 125°C erwärmt. Nach dem Abkühlen wird auf 500 mL 2N Salzsäure gegeben und die wässr. Phase 3 x mit 200 mL EE extrahiert. Die w. Phase wird unter pH- und Temperatur-Kontrolle (20-25°C) mit wässriger NaOH (20%) auf pH 10 gebracht und das als Feststoff anfallende 5-(4-Benzyl-1-piperazinyl)-benzofuran-2-carboxamid abfiltriert. Es wird z.B. aus Ethanol/ Wasser kristallisiert (Auswaage: 4,0 g / 64 % / Fp. 277-279°).

### 3) Synthese von 5-(1-Piperazinyl)-benzofuran-2-carboxamid aus 5-(4-Benzyl-1-Piperazinyl)-benzofuran-2-carboxamid

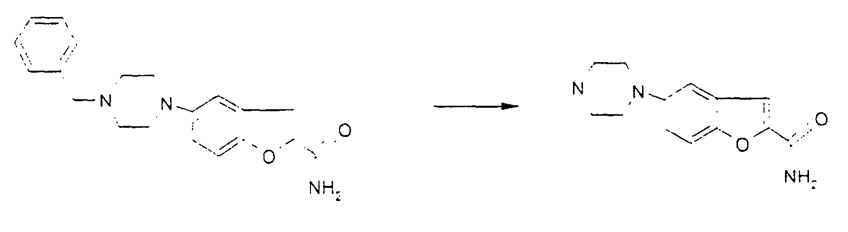

### Hydrogenolyse-Durchführung:

Zu 300 mL Ethanol werden 5,0 g 5-(4-Benzyl-1-Piperazinyl)-benzofuran-2-carboxamid gegeben und nach Zusatz von 9 g Palladium auf Aktivkohle (5%) und 5 g HOAc (100%) bei 20-30 °C erschöpfend mit Wasserstoff debenzyliert. Das Produkt kann nach Filtration und Entfernen des Lösungsmittels im Vakuum und Kristallisation aus Alkohol oder Wasser und Trocknen bei 60 °C im Vakuum isoliert werden (3,1 g / 85%/ Fp. 252-255°. spektroskopisch identisch mit über vorherige Wege gefertigtem Material; beschrieben u.a. in DE 4101686 / Offenleg. 23.7.92; DE 4333254 / Offenleg. 6.4.95; EP 0648767 / Veröff. 19.4.95; EP 0738722 / Veröff. 23.10.96).

### Beispiel 2

### 1) Synthese von 5-(4-tert-Butoxycarbonyl-1-piperazinyl)-benzofuran-2-carboxamid aus 5-Brom-benzofuran-2-carboxamid

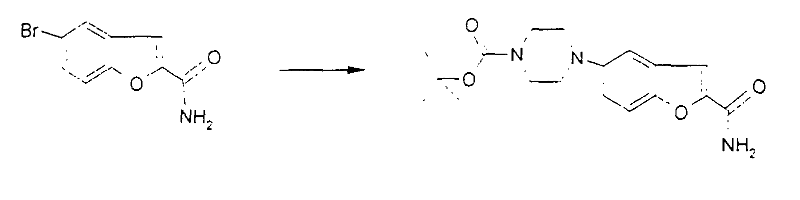

Zu einer Suspension von 0,06 g Pd(DBA)₂ und 0,25 g P(t-Bu)₃ werden in 40 mL Diethylenglycoldimethylether 0.9 g 5-Brom-benzofuran-2-carboxamid, 1.1 g BOC-Piperazin, und 1,45 g Na-t-OBu gegeben und unter Schutzgas für 16 h auf 120-130°C erwärmt. Nach dem Abkühlen wird auf Wasser gegeben und die org. Phase mit 100 mL MTBE verdünnt + mit 3x 50 mL Wasser gewaschen. Das Solvenz wird evaporiert und das als Feststoff anfallende Produkt abfiltriert und mittels Kristallisation aus Ethanol gereinigt (Auswaage: 0,7 g / 55% / Fp. 210-213).

Die sich anschließende, nachfolgend nur als Reaktionsgleichung dargestellte salzsaure Abspaltung der BOC-Schutzgruppe und Bildung von 5-(1-Piperazinyl)-benzofuran-2-carboxamid kann z.B. wie in GREENE T.W. und WUTS P.G.M.. PROTECTIVE GROUPS IN ORGANIC SYNTHESIS beschrieben, erfolgen.

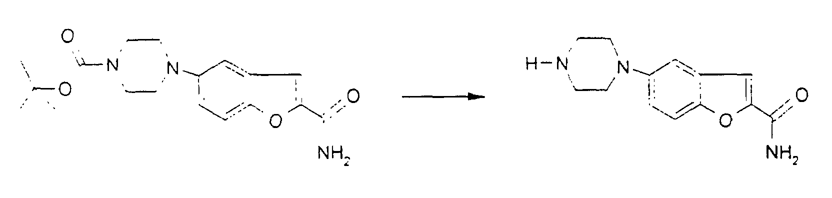

### Beispiel 3

### Synthese von 5-(1-Piperazinyl)-benzofuran-2-carboxamid aus 5-Brom-benzofuran-2-carboxamid

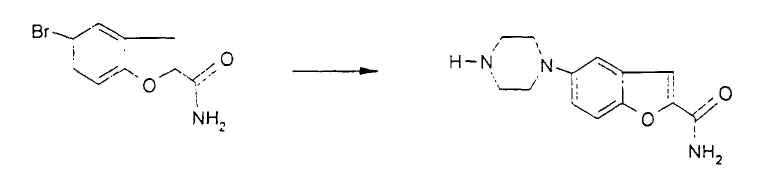

Zu einer Suspension von 0,06 g Pd(DBA)₂ und 0,07 g 1-(N,N-Dimethylamino)-1'-(dicyclohexylphosphino)biphenyl werden in 50 mL Toluol 0.9 g 5-Brom-benzofuran-2-carboxamid, 0,97 g Piperazin, und 2,20 g Na-t-OBu gegeben und unter Schutzgas für 16 h auf 120-130° erwärmt. Nach dem Abkühlen wird das Reaktionsgemisch auf eine Mischung aus 50 ml Wasser und 10 ml 37%iger Salzsäure gegeben, 100 ml Ethylacetat addiert und 20 min gerührt. Anschließend entfernt man wenig ungelöstes Produkt und trennt die organische Phase ab.
Die wässrige Phase wird nochmals mit 50 ml Ethylacetat ausgeschüttelt und im Vakuum von Lösungsmittelresten befreit, mit Kohle geklärt und filtriert. Aus dem Filtrat fällt man bei 20 -22° mit 20 - 25 mL 32%iger Natronlauge das Produkt kristallin aus. Es wird abfiltriert und getrocknet. (Auswaage: 0,65 g / 70 % / Fp. 252-255°).

### Beispiel 4

### 1) Synthese von 5-(4-Benzylpiperazin-1-yl)-2-hydroxybenzaldehyd

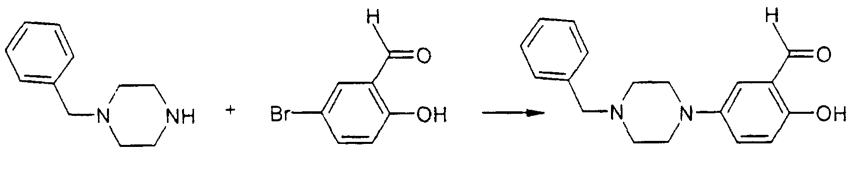

Zu 200 mL Toluol werden unter Stickstoff 0,6 g Bis-(Dibenzylidenaceton)-Palladium und 0,16 g Tri-tert.-butylphosphin gegeben und die entstehende dunkelrote Lösung für 20 min bei 20° gerührt. Es folgt die Zugabe von 10 g 5-Brom-2-hydroxybenzaldehyd, 9,7 g 1-Benzylpiperazin und 7,2 g Natrium-tert.-butylat. Nach 24-stündigem Rühren bei 60° wird abgekühlt, mit 800 mL Wasser versetzt und mit 2 x 500 mL Ethylacetat extrahiert. Die organischen Phasen werden vereinigt, mit 300 mL Wasser gewaschen und im Vakuum bei 30° das Solvenz entfernt. Das verbleibende dunkelorange gefärbte Öl (9,7 g) wird chromatographisch aufgereinigt (300 g Kieselgel; MTB-Ether/Heptan 5:1; 1,5 Liter). Es verbleiben 9,9 g blaßgelbe Kristalle (67%),
F. 101-103°; MS 296 (M+), 205, 119, 91 (100).

### 2) Synthese von 4-(4-Benzyl-piperazin-1-yl)-2-formyl-phenyloxy-essigsäureethylester

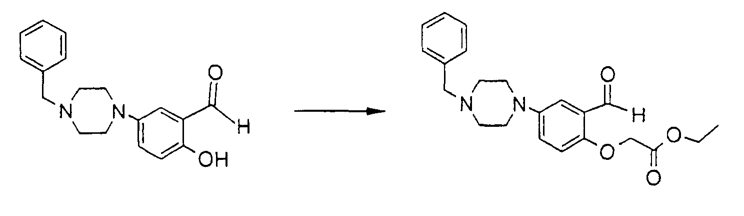

Unter Rühren werden 0,5 g 5-(4-Benzylpiperazin-1-yl)-2-hydroxybenzaldehyd unter Stickstoff bei 20°C in 5 mL NMP gelöst und 0,25 g Kaliumcarbonat und 0,2 mL Ethylbromacetat zugegeben. Es wird 4 Stunden bei 110° gerührt und auf 15° abgekühlt. Die Mischung wird mit 30 mL Wasser und 30 mL Ethylacetat versetzt und nach der Phasentrennung die wässrige Phase mit 30 mL Ethylacetat extrahiert. Die vereinigte organische Phase wird mit 2 x 30 mL Wasser gewaschen und im Vakuum vom Solvenz befreit. Das verbleibende gelbes Öl (0,7 g) wird an 10 g Kieselgel chromatographiert (MTB-Ether/Heptan 5: 1) und liefert 0,45 g Produkt (70%; gelbliches Öl),
MS 382 (M+), 296, 263, 199, 149, 119, 91 (100%).

### Beispiel 5

### 1) Synthese von 5-(4-Benzyl-piperazin-1-yl)-benzofuran-2-carbon-säureethylester

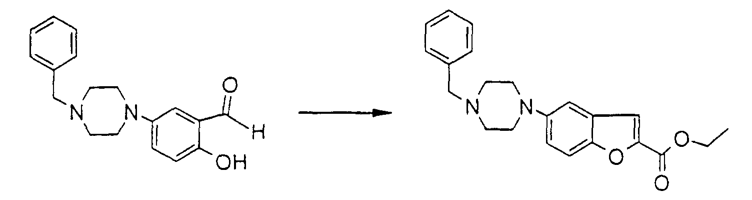

Zu 5 mL NMP werden unter Rühren bei 20° 0,5 g 5-(4-Benzylpiperazin-1-yl)-2-hydroxybenzaldehyd gegeben und die Lösung mit 0,25 g Kaliumcarbonat und 0,2 mL Ethylbromacetat versetzt. Es wird 15 Stunden bei 105° gerührt, dann auf 25° abgekühlt. Der Ansatz wird unter Rühren auf 30 mL Wasser (10°) gegeben, die wässrige Phase bei 10° mit 3 x 50 mL Ethylacetat extrahiert, die vereinigten organischen Phasen mit 50 mL Wasser gewaschen und dann im Vakuum vom Solvenz befreit (1,2 g oranges Öl). Säulenchromatographie an 30 g Kieselgel (MTB-Ether / Heptan 5:1) liefert 0,43 g blaßgelbe Kristalle (71 %),
F. 105-107°; MS 364 (M+). 268, 204, 146, 119, 91 (100%)
Durch Lösen in Ethanol. Zugabe von wässriger 1 N Salzsäure, Isolierung des anfallenden Feststoffes und Trocknen im Vakuum kann ein Muster des entsprechenden Hydrochlorides gewonnen werden (F. 219-222°).

### Beispiel 6

### 1) Synthese von 5-(4-Benzyl-piperazin-1-yl)-benzofuran-2-carboxamid

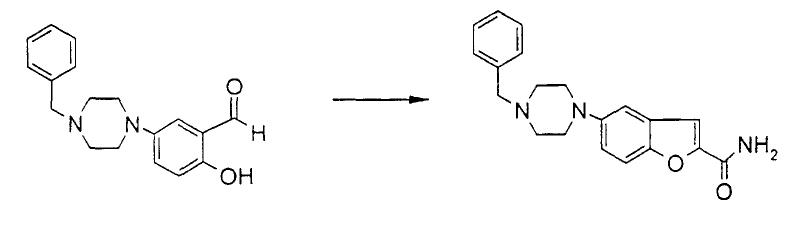

Zu 5 mL NMP werden unter Stickstoff bei 20° 500 mg 5-(4-Benzylpiperazin-1-yl)-2-hydroxybenzaldehyd unter Rühren gegeben und die Lösung mit 0.25 g Kaliumcarbonat und 0.2 mL Ethylbromacetat versetzt.
Es wird 15 Stunden bei 105° gerührt und auf 25° abgekühlt. Hierauf wird die Mischung mit 0,2 mL Formamid versetzt und 30 Minuten nachgerührt.
Es wird dann in 15 Minuten bei 25° 1 mL Natriummethylat (30%ige Lösung in Methanol) zugesetzt und weitere 3 Stunden bei 25-30° gerührt. Die Reaktionsmischung wird auf 30 mL Wasser (10°) gegossen, die wässrige Phase bei 10° mit 3 x 50 mL Ethylacetat extrahiert, die vereinigten organischen Phasen mit 50 mL Wasser gewaschen und im Vakuum das Solvenz entfernt (0,7 g oranges Öl). Es wird aus 10 mL Toluol umkristallisiert (375 mg blaßgelbe Kristalle; 66%), F. 206-208°; MS 335
(M+), 244, 189. 146, 91 (100%).

Durch Abspaltung der Schutzgruppe erhält man 5-(1-Piperazinyl)-benzofuran-2-carboxamid.

### Beispiel 7

### 1) Synthese von 5-(4-tert.Butoxycarbonylpiperazin-1-yl)-2-hydroxybenzaldehyd

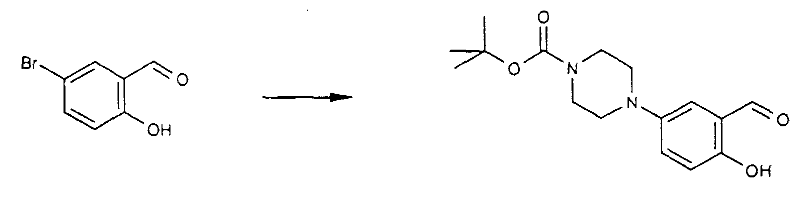

Zu 200 mL Toluol werden unter Stickstoff 0,58 g Bis-(Dibenzylidenaceton)-Palladium und 0,16 g Tri-tert.-butylphosphin gegeben und die entstehende, sich dunkelrot färbende Lösung für 30 min bei 20° gerührt. Es folgt die Zugabe von 10 g 5-Brom-2-hydroxybenzaldehyd, 10,2 g tert.-Butyl-1-piperazincarboxylat und 7,2 g Natrium-tert.-butylat. Nach 24-stündigem Rühren bei 60° wird abgekühlt, mit 800 mL Wasser versetzt und mit 2 x 500 mL Ethylacetat extrahiert. Die organischen Phasen werden vereinigt, mit 300 mL Wasser gewaschen und im Vakuum bei 30° das Solvenz entfernt. Das verbleibende dunkel oranges Öl (11g) wird mittels Chromatographie (300 g Kieselgel; MTB-Ether/Heptan 5:1; 1,5 Liter) aufgereinigt. Es verbleiben 7,8 g blaßgelbe Kristalle (51%), F. 84-86°;
MS 306 (M+), 250 (100%). 233, 176, 164.

### 2) Synthese von 4-(4-tert.-Butoxycarbonylpiperazin-1-yl)-2-formyl-phenyloxy-essigsäureethylester

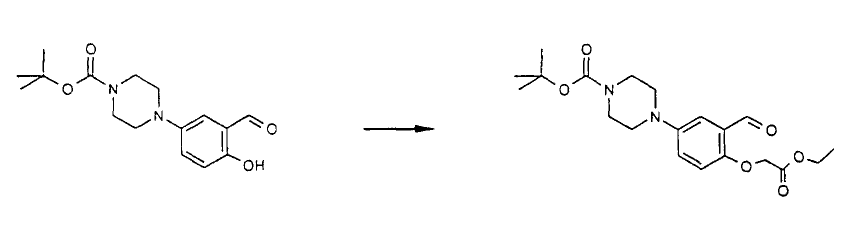

Unter Rühren werden unter Stickstoff 0,5 g 5-(4-tert.-Butoxypiperazin-1-yl)-2-hydroxybenzaldehyd bei 20° in 5 mL NMP gelöst und 0,25 g Kaliumcarbonat und 0,2 mL Ethylbromacetat zugegeben. Es wird 30 Minuten bei 110° gerührt und auf 25° abgekühlt. Die Mischung wird mit 30 mL Wasser und 30 mL Ethylacetat versetzt und nach Phasentrennung die wässrige Phase mit 30 mL Ethylacetat extrahiert. Die vereinigte organische Phase wird mit 30 mL Wasser gewaschen und im Vakuum vom Solvenz befreit. Der verbleibende Kristallbrei wird mit 30 mL Toluol. 30 mL Wasser und 5 mL 1 N HCl versetzt, die Toluolphase im Vakuum entfernt, der kristalline Niederschlag abgetrennt und im Vakuum bei 40° getrocknet (0,48 g; 75%),
F. 93-94°C; MS 392 (M+), 336(100%), 250/249, 57.

### Beispiel 8

### 1) Synthese von 5-(4-tert.-Butoxycarbonyl-piperazin-1-yl)-benzofuran-2-carbonsäureethylester

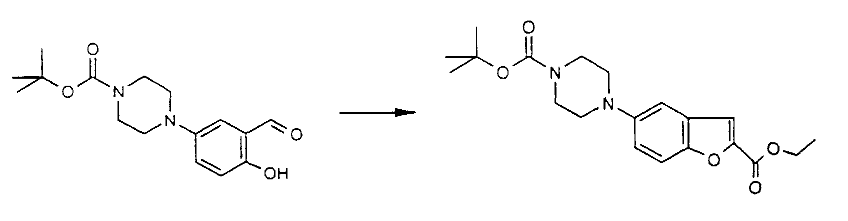

Zu 5 mL NMP werden unter Stickstoff bei 20° unter Rühren 520 mg 5-(4-tert.-Butoxycarbonylpiperazin-1-yl)-2-hydroxybenzaldehyd gegeben und die Lösung mit 0,25 g Kaliumcarbonat und 0,2 mL Ethylbromacetat versetzt. Es wird 3 Stunden bei 105° gerührt, dann auf 25° abgekühlt. Der Ansatz wird unter Rühren auf 30 mL Wasser (10°) gegeben, die wässrige Phase bei 10° mit 3 mal 30 mL Ethylacetat extrahiert, die vereinigten organischen Phasen mit 30 mL gesättigter NaCl-Lösung und mit 30 mL Wasser gewaschen und dann im Vakuum vom Solvenz befreit (0,6 g oranges Öl mit Kristallanteilen). Nach Chromatographie an 30 g Kieselgel (MTB-Ether / Heptan 5:1) sind 0,45 g blaßgelbe Kristalle isolierbar (70 %).
F. 116-117°; MS 374 (M+), 318 (100%), 244, 232.

### Beispiel 9

### 1) Synthese von 5-(4-tert.-Butoxycarbonyl-piperazin-1-yl)-benzofuran-2-carboxamid

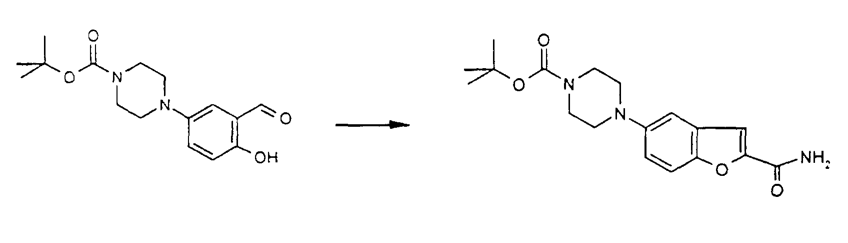

Zu 10 mL NMP werden unter Rühren bei 20° unter Stickstoff 1,04 g 5-(4-tert.-Butoxycarbonylpiperazin-1-yl)-2-hydroxybenzaldehyd gegeben und die Lösung mit 0,5 g Kaliumcarbonat und 0,4 mL Ethylbromacetat versetzt. Es wird 5 Stunden bei 120° gerührt und auf 25° abgekühlt. Hiernach wird die Mischung mit 0,4 mL Formamid versetzt und 30 Minuten nachgerührt. Hierauf wird in 15 Minuten ohne Kühlung 1,9 mL Natriummethylat (30%ige Lösung in Methanol) zugesetzt und eine weitere Stunde bei 25-30° gerührt. Der Ansatz wird mit 30 mL Wasser 30 mL Ethylacetat versetzt und nach Phasentrennung die wässrige Phase mit 30 mL Ethylacetat extrahiert. Die vereinigten organischen Phasen werden mit 30 mL Wasser gewaschen und im Vakuum das Solvenz entfernt (1,1 g oranger Kristallbrei). Nach Kristallisation mit 20 mL Toluol verbleiben 500 mg hellbeige Kristalle. Die Mutterlauge wird eingeengt und das verbleibende Öl mit 10 mL Toluol gelöst. Bei 0° bilden sich nach 3 Stunden weitere hellbeige Kristalle (identisch mit ersten Kristallen; 70 mg). Die gesamte Ausbeute (0,57 g) liegt bei 49 %, F. 202-204°; MS 345 (M+), 289 (100%), 272, 244, 215, 203.
Die Abspaltung der BOC-Gruppe erfolgt wie beschrieben und man erhält 5-(1-Piperazinyl)-benzofuran-2-carboxamid.

### Beispiel 10

### 1) Synthese von 5-(4-tert.-Butoxycarbonyl-piperazin-1-yl)-benzofuran-2-carboxamid

Zu 0,5 g 5-(4-tert.-Butoxycarbonylpiperazin-1-yl)-2-hydroxybenzaldehyd werden bei 20 °C unter Rühren / unter Stickstoff 5 mL 1-Methyl-2-Pyrrolidon, 0,16 g Chloracetamid und 0,25 g Kaliumcarbonat gegeben. Es wird 16 Std bei 60 °C gerührt, dann nach Abkühlen filtriert und das Solvenz im Vakuum entfernt. Der Rückstand wird in MTB-Ether aufgenommen, erneut filtriert. aufkonzentriert und der Rückstand aus Toluol kristallisiert. Die isolierte Ausbeute beträgt 0.34 g (60 %).

### 2) Synthese von 5-(4-Benzyl-piperazin-1-yl)-benzofuran-2-carboxamid

Zu 1,0 g 5-(4-Benzylpiperazin-1-yl)-2-hydroxybenzaldehyd werden bei 20 °C unter Rühren / unter Stickstoff 10 mL 1-Methyl-2-Pyrrolidon, 0,4 g Chloracetamid und 0.8 g Kaliumcarbonat gegeben. Es wird 16 Std bei 60 °C gerührt, dann nach Abkühlen filtriert und das Solvenz im Vakuum entfernt. Der Rückstand wird in MTB-Ether aufgenommen, erneut filtriert, aufkonzentriert und der Rückstand aus Toluol kristallisiert. Die isolierte Ausbeute beträgt 0,73 g (65 %).

## Patentansprüche

1. Verfahren zur Herstellung von 5-(1-Piperazinyl)-benzofuran-2-carboxamid und/oder eines seiner Salze, **dadurch gekennzeichnet, daß** man
a) 5-Brom-salicylaldehyd in einer Eintopfreaktion zuerst mit einer Verbindung der Formel I
L-CH₂-COOR¹ I
worin
L Cl, Br, l oder eine reaktionsfähig veresterte OH-Gruppe und
R¹ Alkyl mit 1-6 C-Atomen oder Benzyl bedeuten,
und anschließend mit Formamid zu 5-L-benzofuran-2-carboxamid (II),
worin L Cl, Br, l oder eine reaktionsfähig veresterte OH-Gruppe bedeutet,
umsetzt,
darauf (II) in einer Übergangsmetall-katalysierten Aminierung mit R²-Piperazin, worin R² H oder eine Aminoschutzgruppe bedeutet,
zu einer Verbindung der Formel III 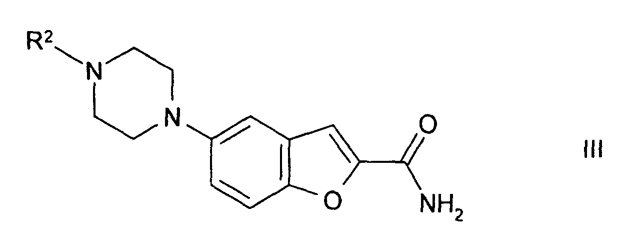 worin R² H oder eine Aminoschutzgruppe bedeutet,
umsetzt,
und anschließend, falls R² ≠ H ist, R² abspaltet,
oder
b) eine Verbindung der Formel IV 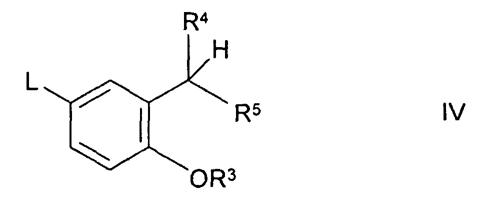 worin
L Cl, Br, I oder eine reaktionsfähig veresterte OH-Gruppe,
R³ H oder CH₂R⁶,
R⁴ und R⁵ zusammen Carbonyl,
R⁶ CN, COOH, COOR⁷ oder CONH₂,
R⁷ Alkyl mit 1-6 C-Atomen,
n 2 oder 3
bedeuten,
in einer Übergangsmetall-katalysierten Aminierung mit R²-Piperazin,
worin R² H oder eine Aminoschutzgruppe bedeutet,
zu einer Verbindung der Formel V 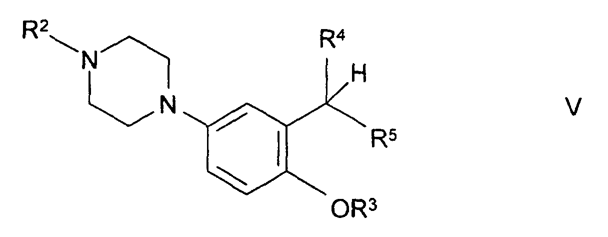 worin
R² H oder eine Aminoschutzgruppe,
R³ H, CH₂R⁶,
R⁴ und R⁵ zusammen Carbonyl,
R⁶ CN, COOH, COOR⁷ oder CONH₂,
R⁷ Alkyl mit 1-6 C-Atomen,
n 2 oder 3
bedeuten,
umsetzt,
und anschließend, falls R² ≠ H ist, R² abspaltet,
oder
c) eine Verbindung der Formel V
worin
R² eine Aminoschutzgruppe,
R³ H, CH₂R⁶,
R⁴ und R⁵ zusammen Carbonyl,
R⁶ CN, COOH, COOR⁷ oder CONH₂,
R⁷ Alkyl mit 1-6 C-Atomen
n 2 oder 3
bedeuten,
mit Chloracetamid zu einer Verbindung der Formel III
worin R² eine Aminoschutzgruppe bedeutet,
umsetzt,
und anschließend R² abspaltet,
und/oder daß man 5-(1-Piperazinyl)-benzofuran-2-carboxamid durch Behandeln mit einer Säure in eines seiner Säureadditionssalze umwandelt.

2. Verfahren nach Anspruch 1, **dadurch gekennzeichnet, daß** in der Verbindung der Formel I L Br bedeutet.

3. Verfahren nach Anspruch 1, **dadurch gekennzeichnet, daß** als Übergangsmetall-Katalysator-System Pd(OAc)₂ / P(tert.-Butyl)₃ verwendet wird.

4. Verfahren nach Anspruch 1, **dadurch gekennzeichnet, daß** bei der Umsetzung von 5-Brom-salicylaldehyd bzw. einer Verbindung der Formel V mit einer Verbindung der Formel I N-Methylpyrrolidon als Lösungsmittel verwendet wird.

5. Verbindungen der Formel V 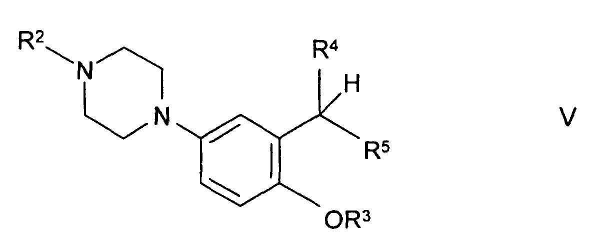 worin
R² H oder eine Aminoschutzgruppe,
R³ H, CH₂R⁶,
R⁴ und R⁵ zusammen Carbonyl,
R⁶ CN, COOH, COOR⁷ oder CONH₂,
R⁷ Alkyl mit 1-6 C-Atomen,
n 2 oder 3
bedeuten,
sowie deren Salze und Solvate.

## Claims

1. Process for the preparation of 5-(1-piperazinyl)benzofuran-2-carboxamide and /or one of its salts, **characterised in that**
a) 5-bromosalicylaldehyde is reacted in a one-pot reaction firstly with a compound of the formula I
L-CH₂-COOR¹ I
in which
L denotes Cl, Br, I or a reactively esterified OH group and
R¹ denotes alkyl having 1-6 C atoms or benzyl,
and subsequently with formamide to give 5-L-benzofuran-2-carboxamide (II),
in which L denotes Cl, Br, I or a reactively esterified OH group,
(II) is then reacted in a transition metal-catalysed amination with R²-piperazine, in which R² denotes H or an amino-protecting group, to give a compound of the formula III 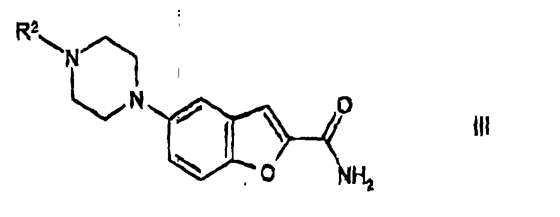 in which R² denotes H or an amino-protecting group,
and subsequently, if R² ≠ H, R² is cleaved off,
or
b) a compound of the formula IV 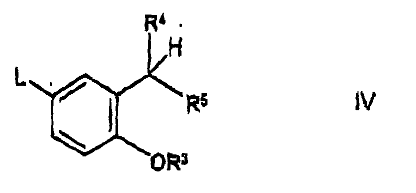 in which
L denotes Cl, Br, I or a reactively esterified OH group,
R³ denotes H or CH₂R⁶,
R⁴ and R⁵ together denote carbonyl,
R⁶ denotes alkyl having 1-6 C atoms,
R⁷ denotes CN, COOH, COOR⁷ or CONH₂,
n denotes 2 or 3,
is reacted in a transition metal-catalysed amination with R²-piperazine, in which R² denotes H or an amino-protecting group, to give a compound of the formula V 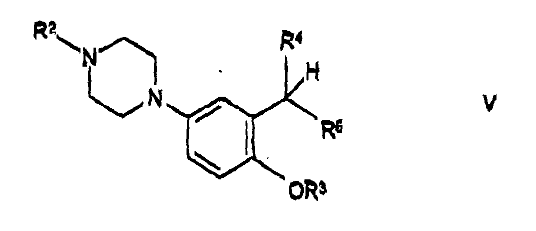 in which
R² denotes H or an amino-protecting group,
R³ denotes H, CH₂R⁶,
R⁴ and R⁵ together denote carbonyl,
R⁶ denotes CN, COOH, COOR⁷ or CONH₂,
R⁷ denotes alkyl having 1-6 C atoms,
n denotes 2 or 3,
and subsequently, if r² ≠ R² is cleaved off,
or
c) a compound of the formula V
in which
R² denotes an amino-protecting group,
R³ denotes H, CH₂R⁶, group,
R⁴ and R⁵ together denote carbonyl,
R⁶ denotes CN, COOH, COOR⁷ or CONH₂,
R⁷ denotes alkyl having 1-6 C atoms,
n denotes 2 or 3,
is reacted with chloroacetamide to give a compound of the formula III
in which R² denotes an amino-protecting group,
and R² is subsequently cleaved off,
and/or **in that** 5-(1-piperazinyl)benzofuran-2-carboxamide is converted into one of its acid-addltion salts by treatment with an acid.

2. Process according to Claim 1, **characterised in that**, in the compound of the formula I, L denotes B.

3. Process according to Claim 1, **characterised in that** the transition-metal catalyst system used is Pd(OAc)₂/P(tert-butyl)₃.

4. Process according to Claim 1, **characterised in that**, in the reaction of 5-bromosalicylaldehyde or a compound of the formula V with a compound of the formula I, N-methylpyrrolidone is used as solvent.

5. Compounds of the formula V. 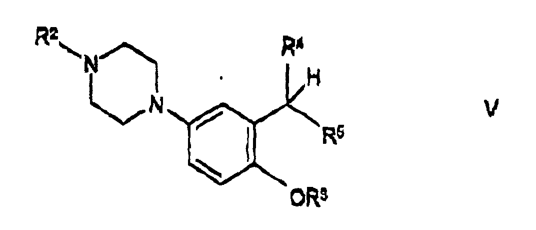 in which
R² denotes H or an amino-protecting group,
R³ denotes H, CH₂R⁶,
R⁴ and R⁵ together denote carbonyl,
R⁶ denotes CN, COOH, COOR⁷ or CONH₂,
R⁷ denotes alkyl having 1-6 C atoms,
n denotes 2 or 3,
and salts and solvates thereof.

## Revendications

1. Procédé pour la préparation de 5-(1-pipérazinyl)benzofurane-2-carboxamide et/ou d'un de ses sels, **caractérisé en ce que**
a) 5-bromosalicylaldéhyde est amené à réagir dans une réaction monocellulaire tout d'abord avec un composé de la formule I
L-CH₂-COOR¹ I
dans laquelle
L représente Cl, Br, I ou un groupe OH estérifié de façon réactive et
R¹ représente alkyle ayant 1-6 atomes de C ou benzyle,
et ensuite avec formamide pour donner 5-L-benzofurane-2-carbox-amide (II),
dans laquelle L représente Cl, Br, I ou un groupe OH estérifié de façon réactive,
(II) est alors amené à réagir dans une amination catalysée par métal de transition avec R²-pipérazine, dans laquelle R² représente H ou un groupe de protection amino,
pour donner un composé de la formule III 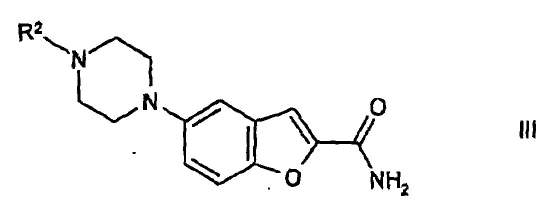 dans laquelle R² représente H ou un groupe de protection amino,
et ensuite, si R² ≠ H, R² est enlevé par séparation,
ou
b) un composé de la formule IV 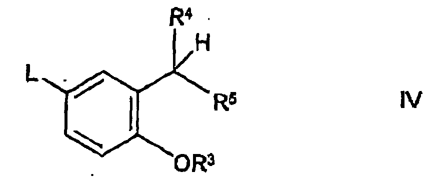 dans laquelle
L représente Cl, Br, I ou un groupe OH estérifié de façon réactive,
R³ représente H ou CH₂R⁶,
R⁴ et R⁵ représentent ensemble carbonyle,
R⁶ représente CN, COOH, COOR⁷ ou CONH₂,
R⁷ représente alkyle ayant 1-6 atomes de C,
n représente 2 ou 3,
est amené à réagir dans une amination catalysée par métal de transition avec R²-pipérazine, dans laquelle R² représente H ou un groupe de protection amino,
pour donner un composé de la formule V 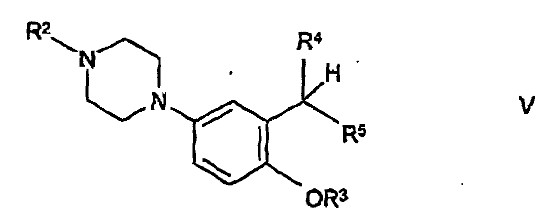 dans laquelle
R² représente H ou un groupe de protection amino,
R³ représente H, CH₂R⁶,
R⁴ et R⁵ représentent ensemble carbonyle,
R⁶ représente CN, COOH, COOR⁷ ou CONH₂,
R⁷ représente alkyle ayant 1-6 atomes de C,
n représente 2 ou 3,
ensuite amené à réagir selon une réaction monocellulaire tout d'abord avec un composé de la formule I
L-CH₂-COOR¹ I
dans laquelle
L représente Cl, Br, I ou un groupe OH estérifié de façon réactive et
R¹ représente alkyle ayant 1-6 atomes de C ou benzyle,
et ensuite avec formamide pour donner un composé de la formule III 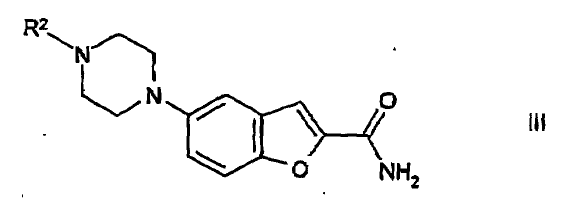 dans laquelle R² représente H ou un groupe de protection amino,
et ensuite, si R² ≠ H, R² est enlevé par séparation,
ou
c) un composé de la formule V
dans laquelle
R² représente un groupe de protection amino,
R³ représente H, CH₂R⁶,
R⁴ et R⁵ représentent ensemble carbonyle,
R⁶ représente CN, COOH, COOR⁷ ou CONH₂.
R⁷ représente alkyle ayant 1-6 atomes de C,
n représente 2 ou 3,
est amené à réagir avec chloroacétamide pour donner un composé de la formule III
dans laquelle R² représente un groupe de protection amino,
et R² est ensuite enlevé par séparation,
et/ou **en ce que** 5-(1-pipérazinyl)benzofurane-2-carboxamide est converti selon un de ses sels par addition d'acide par traitement avec un acide.

2. Procédé selon la revendication 1, **caractérisé en ce que**, dans le composé de la formule I, L représente Br.

3. Procédé selon la revendication 1, **caractérisé en ce que** le système de catalyseur de métal de transition utilisé est Pd(OAc)₂/P(tert-butyl)₃.

4. Procédé selon la revendication 1, **caractérisé en ce que**, dans la réaction de 5-bromosalicylaldéhyde ou un composé de la formule V avec un composé de la formule I, N-méthylpyrrolidone est utilisé en tant que solvant.

5. Composés de la formule V 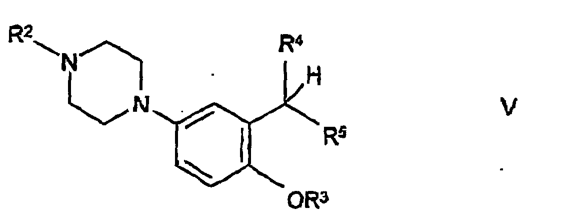 dans laquelle
R² représente H ou un groupe de protection amino,
R³ représente H, CH₂R⁶,
R⁴ et R⁵ représentent ensemble carbonyle,
R⁶ représente CN, COOH, COOR⁷ ou CONH₂,
R⁷ représente alkyle ayant 1-6 atomes de C,
n représente 2 ou 3,
et leurs sels et sovates.
